(19) Europäisches Patentamt
European Patent Office
Office européen des brevets

(11) Publication number: **0 542 107 A1**

(12) **EUROPEAN PATENT APPLICATION**

(21) Application number: **92118833.0**

(22) Date of filing: **03.11.92**

(51) Int. Cl.[5]: **C12Q 1/26**, C12Q 1/28, C12Q 1/54, G01N 33/52, G01N 33/98

(30) Priority: **15.11.91 US 792669**

(43) Date of publication of application: **19.05.93 Bulletin 93/20**

(84) Designated Contracting States: **AT BE CH DE ES FR GB GR IT LI LU NL SE**

(71) Applicant: **MILES INC.**
**One Mellon Center 500 Grant Str.**
**Pittsburgh, PA 15219-2502(US)**

(72) Inventor: **Bauer, Robert**
**56180 CR 21**
**Bristol, Indiana 46507(US)**
Inventor: **Cattell, John A.**
**56269 Silvercrest Drive**
**Elkhart, Indiana 46516(US)**

(74) Representative: **Dänner, Klaus, Dr. et al**
**c/o Bayer AG Konzernverwaltung RP**
**Patentabteilung**
**W-5090 Leverkusen 1 Bayerwerk (DE)**

# (54) Detection of analytes in saliva using peroxide-peroxidase test systems.

(57) Disclosed is a method for the detection of an analyte in saliva by the oxidase, peroxidase redox indicator method. The method involves selecting an indicator which has an oxidation potential low enough to enable it to provide a suitable response under the conditions of the assay.

EP 0 542 107 A1

## Background Of The Invention

The present invention is concerned with a method for the semi-quantitative determination of analytes which generate $H_2O_2$ in the presence of an oxidase selective for such analyte to liberate hydrogen peroxide in saliva samples. The determination of such analytes in body fluids such as blood and urine is exemplified by the system for detecting glucose which contains glucose oxidase, a peroxidatively active substance [POD] and a chromogenic oxidation indicator [Ind] which is oxidized upon exposure to hydrogen peroxide in the presence of the peroxidatively active substance. This system is exemplified by the formation of hydrogen peroxide by the action of glucose oxidase on glucose:

$$\text{Glucose} + O_2 \xrightarrow{\text{[glucose oxidase]}} H_2O_2 + \text{gluconolactone}$$

and the resultant oxidation of the indicator to its oxidized state $[\text{Ind}]_{ox}$ in the presence of a peroxidase which oxidation is visually detectable by a color change:

$$H_2O_2 + [\text{Ind}] \xrightarrow{\text{[POD]}} H_2O + [\text{Ind}]_{ox}$$

A similar system can be used for the detection of ethanol by substituting alcohol oxidase for glucose oxidase. The use of saliva for detection of various analytes, such as glucose, alpha amylase, thiocyanate steroid hormones, ethanol, thyroxine, melatonin and urea is disclosed in Human Saliva:Clinical Chemistry and Microbiology, CRC Press, 1989 (Vol. 1 and 2). Saliva would be a viable body fluid for the quantitative detection of various clinically important analytes such as glucose, lactate, uric acid and particularly ethanol. While color can be generated from these systems by the oxidation of various indicators in the presence of peroxidase, it has been discovered that there exist one or more components in saliva that can reduce the oxidized (colored) form of certain chromogenic indicators to a colorless form.

## Summary Of The Invention

The present invention involves a method for the detection in saliva of analytes which, in the presence of a suitable oxidase enzyme, produce hydrogen peroxide. The method involves combining the saliva with an oxidase selective for the analyte, a peroxidase or a peroxidatively active substance and an indicator which is oxidizable by hydrogen peroxide in the presence of peroxidase to provide a detectable response wherein the indicator is selected from those indicators having sufficiently low oxidation potentials to render them resistant to reducing agents normally present in the saliva and to thereby provide a response which can be detected by colormetric means.

## Description Of The Invention

In its most general terms, the invention is practiced by contacting a sample of human saliva with a reagent system comprising an oxidase selective for the analyte whose presence is being sought, a peroxidase and a redox indicator which will provide a detectable response when oxidized by hydrogen peroxide produced by the interaction between the analyte and the oxidase therefore in the presence of the peroxidase. This mechanism works well when blood or urine are the bodily fluids being tested since the concentration of the analyte will be directly proportional to the intensity of the color formed by oxidation of the oxidizable indicator. However, ascorbate ion which may be present in blood or urine tends to adversely affect the precision of this system. The oxidase/peroxidase test would be particularly suitable for saliva, which contains no ascorbate, however, this system does not work well with saliva because this fluid contains certain reducing agents which tend to interfere with the oxidation of the indicator. This interference tends to manifest itself in the formation of too little color, or, if the appropriate amount of color does form, it tends to fade so quickly that an accurate reading of its intensity cannot be made. This phenomena was observed with many of the commonly employed redox indicators such as tetramethyl benzidine, syringaldazine, o-tolidine and azino-diethylbenzthiazoline. Based on the hypothesis that these indicators do not perform satisfactorily in saliva because of the presence of certain unspecified reducing agents being contained therein, it was decided to try using indicators with lower oxidation potentials in the hope that they

would be resistant to reduction by those unidentified reducing agents typically present in saliva. Included among these redox indicators which typically have an oxidation potential lower than about 0.54 are gum guaiac m-anisidine, p-amino dimethylanaline, and leuco methylene blue. Also included within the scope of those indicators which are suitable for use in the present system are the coupling type indicators, i.e., an indicator consisting of two compounds, one of which is oxidized by the hydrogen peroxide in the presence of peroxidase and this oxidized form of the compound then covalently couples with the second compound to provide a colorimetrically detectable response. Examples of such indicator systems which are suitable for use in the analysis of saliva are methyl benzothiazolinone hydrazone/dimethylaminobenzoic acid; 4-amino-antipyrine/chromotopic acid; 4-amino-antipyrine/primaquine diphosphate; chicago acid/4-aminoantipyrine and 4-aminoantipyrine/4-methylcatechol.

In addition to the indicator, the reagent system requires the presence of an oxidase selective for the analyte under investigation. Thus, if one were testing for glucose, glucose oxidase would be used, for galactose, galactose oxidase, lactic acid oxidase when lactic acid is the analyte of interest and so forth. Galactose is apparently not secreted by the salivary glands but is present from the breakdown of lactose in the mouth and can contribute to plaque formation. Lactic acid in saliva generally correlates well with serum lactate and a quick means for its detection would be useful to gage the degree of exertion during sporting events. The present system is especially suitable for the detection of ethanol in saliva because of the close relationship between blood alcohol and saliva alcohol levels, in which case alcohol oxidase would be incorporated into the reagent system.

In addition, the reagent system includes a material having peroxidase activity to catalyze the oxidation of the redox indicator by the hydrogen peroxide generated by the action of the oxidase enzyme on the analyte. Examples of suitable materials are hemoglobin, molybdate/iodide, ferric EDTA, horseradish peroxidase, lactoperoxidase and myeloperoxidase.

The reagent system can be used in either a wet or dry format. For so-called wet chemistry analysis, the test composition is used as a liquid or as a solid which is dissolvable in water or another suitable solvent to provide a reagent solution. Where the reagent consists of individual components, such can be mixed together to form a final reaction volume. After mixing the saliva sample with an aliquot of the reagent, the resulting color is measured in a photometer and the respective analyte concentration is calculated by means of the molar extinction coefficients and the reagent or sample volumes added.

The reagent composition can also be incorporated with a carrier matrix in the form of a reagent strip. Suitable carrier matrices are known in the art and include absorbent papers, woven and nonwoven cloth, glass fiber filters, polymeric membranes and films. Incorporation methods include impregnation of a formed carrier matrix with a solution, suspension, or other liquid form of the test composition, in one or more steps, followed by drying of the matrix; and formation of a matrix in the presence of one or more of the components of the test composition, e.g., by casting or layering solutions of film or membrane forming formulations. Absorbent or swellable carriers such as filter paper or absorbent glass fiber filters or synthetic nonwovens are impregnated or sprayed with these solutions and then dried. The test composition can also be incorporated with carrier matrices which have been prepared from casting solutions. Cellulose, cellulose derivatives, gelatin, gelatin derivatives or, alternatively, porous plastics such as polyurethane and acrylamide may be mentioned by way of example.

Typically, the concentrations of the various elements of a reagent system suitable for the determination of ethanol in saliva will range from about 10 to 250 IU (International Units)/mL alcohol oxidase, 24 to 3600 IU/mL of peroxidase and 20 to 50 mM for the redox indicator. The requirements for other oxidase reagent systems will vary depending on the turnover rate of the enzyme and the upper limit of analyte concentration range. This solution can either be used as is in a wet reagent test or applied to the reagent strips previously described in the production of the dry test strips.

The present invention is further illustrated by the following examples in which the indicators used are abbreviated as follows:

| | |
|---|---|
| ABTS | = 2,2'-azino-bis-(3-ethylbenzthiazoline-6-acid) |
| MBTH-DMAB | = methyl benzothiazolinone hydrazone/dimethylaminobenzoic acid |
| AAP-DMAB | = 4-aminoantipyrine/dimethylaminobenzoic acid |
| AAP-PDP | = 4-aminoantipyrine/primaquine diphosphate |
| TMB | = 3,3',5,5'-tetramethylbenzidine |

**Example I**

A reagent solution was prepared having the following composition:

| | |
|---|---|
| phosphate, 0.2 M, pH 7.5 | 24.5 mL |
| indicator | 0.1 millimole |
| peroxidase | 1.0 mg |
| alcohol oxidase, 525 U/mL | 0.5 mL |
| TOTAL | 25.0 mL |

Assays were carried out on a Gilford response spectrophotometer at the absorbance maximum for the indicator using the following recipe:

| | |
|---|---|
| 1.5 mL | reagent |
| 1.0 mL | $H_2O$ |
| 0.5 mL | saliva |
| 50 µL | ethanol, 25 mg/dL in $H_2O$ |

Using this general procedure several indicators were evaluated using 7 or 8 individual saliva samples. The results are summarized in the following Table 1.

TABLE 1

| Indicators | Number of Saliva Samples | Apparent EtOH Concentration mg/dL | C.V. |
|---|---|---|---|
| ABTS | Control ($H_2O$) | 25 | – |
| ABTS | 8 | 11 ± 7 | 64% |
| MBTH – DMAB | 8 | 21 ± 2 | 10% |
| AAP – DMAB | 8 | 27 ± 8 | 30% |
| AAP – PDP | 7 | 20 ± 5 | 25% |

The indicator ABTS which, in the oxidized form is decolorized by saliva, showed an apparent ethanol concentration substantially less than the expected 25 mg/dL and significant scatter among the 8 saliva samples as indicated by the high coefficient of variation value. The other indicators which are not decolorized by saliva exhibited ethanol values near the expected value and much less scatter.

**Example II**

A second experiment was carried out with test strips containing either TMB or MBTH – DMAB indicator. These strips which used S + S 593c filter paper as the support were prepared as follows:

TABLE 2

| First Dip | 0.05 M TMB in toluene | 0.05 M MBTH in ethyl acetate |
|---|---|---|
| Second Dip | | |
| Emulphor ON 870, 10% | 2.13 mL | 2.13 mL |
| Sucrose, 80% | 3.12 | 3.12 |
| Phosphate, 2.0 M, pH 8.0 | 2.75 | 2.75 |
| Alcohol oxidase, 525 U/ml | 7.15 | 7.15 |
| $NaN_3$, 0.05 M | 1.37 | 0.75 |
| Peroxidase, 80 mg/mL | 0.78 | 0.78 |
| DMAB | -- | 206 mg |
| $H_2O$ | 7.70 | 8.32 |
| TOTAL | 25.0 | 25.0 |

The impregnated strips were dried at 60 ° C for 3 minutes after the first (indicator in organic solvent) dip and at 70 ° C for 6 minutes after the second (enzyme, buffer, surfactant, stabilizer) dip

The strips were dipped into five individual saliva samples containing 60 mg/dL ethanol. The results are set out in the following Table 3.

Table 3

| | (TMB) | (MBTH – DMAB) |
|---|---|---|
| Apparent EtOH, mg/dL | 43 | 56 |
| Standard Deviation | 14 | 4 |
| C.V. | 33% | 7% |

The MBTH – DMAB strip demonstrated substantially less scatter than did the strips using TMB indicator while indicating an apparent ethanol level which was much closer to the actual ethanol content of saliva.

**Example III**

The oxidation potentials of several redox indicators were obtained from Clark, W.M., Oxidation – Reduction Potentials of Organic Systems, Williams and Wilkins Co. (1960).

In a system buffered at pH 7.0, the oxidized (colored) forms of several indicators were treated with saliva. Any loss or diminished color resulting from the saliva was noted. The results are set out in the following table.

TABLE 4

| Indicator | Observation | Oxidation Potential |
|---|---|---|
| o-Tolidine | Decolorized | +0.54 |
| p-Amino Dimethylanaline | No Decoloration | +0.38 |
| 2,6-Dichlorophenyliodophenol | No Decoloration | +0.217 |
| Gallocyanine | No Decoloration | +0.021 |
| Leuco Methylene Blue | No Decoloration | +0.011 |

The oxidation potentials presented in Table 3 were taken at pH 7.0 at 50% standard as per the standard procedure. The convention used by Clark is that the lower the number, the more difficult it is to reduce the indicator's oxidized form. Thus, according to this experiment the oxidized (colored) form of o-tolidine would be more readily reduced by the reducing substances in saliva than would the oxidized forms of the other indicators with the critical potential lying between +0.54 and +0.38.

## Claims

1. A method for the detection of an analyte which generates hydrogen peroxide in the presence of a suitable oxidase in saliva which method comprises contacting the saliva with a reagent system comprising an oxidase selective for the analyte to be detected, a peroxidatively active material and a redox indicator said indicator being selected from those indicators which have an oxidation potential which is sufficiently low to resist reduction by reducing agents present in the saliva.

2. The method of claim 1 wherein the analyte is glucose, lactic acid, galactose or ethanol.

3. The method of any of claims 1 and 2 wherein the indicator has an oxidation potential less than 0.54.

4. The method of any of claims 1 to 3 wherein the indicator is gum guaiac, m-anisidine, p-amino dimethylanaline, or leuco methylene blue, 2,6-dichlorophenol-iodophenol or gallocyanine.

5. The method of any of claims 1 to 3 wherein the indicator is of the coupling type.

6. The method of claim 5 wherein the coupling indicator is methyl benzothiazoline hydrazone/-dimethylaminobenzoic acid; 4-aminoantipyrine primaquine diphosphate or 4-aminoantipyrine/dimethyl aminobenzoic acid.

7. The method of any of claims 1 to 6 wherein the reagent system is incorporated into a carrier matrix.

8. A method for the detection of alcohol in human saliva which comprises contacting said saliva with a reagent system according to any of claims 1 to 6.

9. The method of claim 8 wherein the reagent system is incorporated into a matrix system to provide a reagent strip.

European Patent Office

# EUROPEAN SEARCH REPORT

Application Number

EP 92 11 8833

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 164 008 (MILES INC.)<br>* the whole document *<br>--- | 1-9 | C12Q1/26<br>C12Q1/28<br>C12Q1/54<br>G01N33/52<br>G01N33/98 |
| A | DATABASE WPIL<br>Section Ch, Week 9033,<br>Derwent Publications Ltd., London, GB;<br>Class B04, AN 90-251367<br>& JP-A-2 176 565 (KONICA CORP.) 9 July 1990<br>* abstract *<br>--- | 1-9 | |
| A | DATABASE WPIL<br>Section Ch, Week 8732,<br>Derwent Publications Ltd., London, GB;<br>Class B04, AN 87-225141<br>& JP-A-62 150 166 (AGENCY OF IND. SCI. TECHN.; AMANO PHARM. KK) 4 July 1987<br>* abstract *<br>--- | 1-9 | |
| A | DATABASE WPIL<br>Section Ch, Week 8902,<br>Derwent Publications Ltd., London, GB;<br>Class B04, AN 89-013539<br>& JP-A-63 291 598 (MEIDENSHA ELEC. MFG. KK) 29 November 1988<br>* abstract *<br>----- | 1-6 | TECHNICAL FIELDS SEARCHED (Int. Cl.5 )<br>C12Q<br>G01N |

The present search report has been drawn up for all claims

| Place of search | Date of completion of the search | Examiner |
|---|---|---|
| THE HAGUE | 24 FEBRUARY 1993 | DöPFER K.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)